# EUROPEAN PATENT SPECIFICATION

(11) **EP 2 493 864 B1**
(45) Date of publication and mention of the grant of the patent: **01.10.2014**
(21) Application number: 10771744.9
(22) Date of filing: 29.10.2010
(51) Int. Cl.: C07D 239/48, A61K 31/506, A61P 35/00

(54) **N-OXIDE OF 3-(2,6-DICHLORO-3,5-DIMETHOXY-PHENYL)-1-{6-[4-(4-ETHYL-PIPERAZIN-1-YL)-PHENYLAMINO]-PYRIMIDIN-4-YL}-1-METHYL-UREA**
N-Oxid von 3-(2,6-Dichlor-3,5-dimethoxyphenyl)-1-{6-[4-(4-ethylpiperazin-1-yl)phenylamino]pyrimidin-4-yl}-1-methylharnstoff
N-oxyde de 3-(2,6-dichloro-3,5-diméthoxy-phényl)-1-{6-[4-(4-éthyl-pipérazin-1-yl)-phénylamino]-pyrimidin-4-yl}-1-méthyl-urée

(30) Priority: 30.10.2009 EP 09174619
(43) Date of publication of application: 05.09.2012
(73) Proprietor: Novartis AG, 4056 Basel (CH)
(72) Inventor: AICHHOLZ, Reiner, CH-4002 Basel (CH); BLASCO, Francesca, CH-4002 Basel (CH); BORDAS, Vincent, CH-4002 Basel (CH); GRAUS PORTA, Diana, CH-4002 Basel (CH); GUAGNANO, Vito, CH-4002 Basel (CH)
(74) Representative: Pfister-Fu, Yixin
(86) International application number: PCT/EP2010/066435
(87) International publication number: WO 2011/051425

(56) References cited:
- WO-A-2007/071752

## Description

### FIELD OF INVENTION

The present technology relates to a novel N-oxide of 3-(2,6-dichloro-3,5-dimethoxy-phenyl)-9-{6-[4-(4-ethyl-piperazin-1-yl)-phenylamino]-pyrimidin-4-yl}-1-methyl-urea, methods for its preparation, compositions containing it, and methods of treatment employing it.

### BACKGROUND OF THE INVENTION

3-(2,6-Dichloro-3,5-dimethoxy-phenyl)-1-{6-[4-(4-ethy)-piperazin-1-yl)-phenylamino]-pyrimidin-4-yl}1-methyl-urea has the following structure:

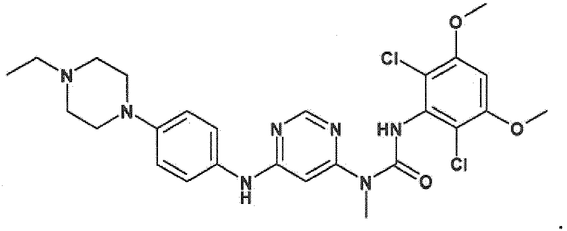

The compound of Formula I is a protein kinase inhibitor and is useful in the treatment of proliferative diseases mediated by protein kinases. In particular, the compound of Formula I inhibits fibroblast growth factor receptor (FGFR) tyrosine kinases. It is therefore useful in the treatment of certain cancers in which FGFR are implicated including breast cancer, gastric cancer, lung cancer, cancer of the prostate, bladder cancer and endometrial cancer.

WO 2007/071752 discloses a broad family of compounds of Formula (IA) and their salts, prodrug, N-oxide or an ester (claim 1). The compound of example 20 in WO 2007/071752 differs from the present compound of formula I in that it is not in it's N-oxide form. N-oxide of compound of example 20 is not disclosed in WO 2007/071752.

### SUMMARY OF THE INVENTION

There is provided herein an N-oxide of 3-(2,6-Dichloro-3,5-dimethoxy-phenyl)-1-{6-[4-(4-ethyl-piperazin-1-yl)-phenylamino]-pyrimidin-4-yl}-1-methyl-urea (which may be prepared, for example, by oxidizing 3-(2,6-Dichloro-3,5-dimethoxy-phenyl)-1-{6-[4-(4-ethyl-piperazin-1-yl)-phenylamino]-pyrimidin-4-yl}-1-methyl-urea with an oxidizing agent, such as, mCPBA), compositions including the compound, and methods of preparing the compound and compositions. The present technology further provides methods of using the compound and compositions of the present technology to treat various diseases, alone and in combination with other suitable agents, including but not limited to, those that can be prevented, inhibited or ameliorated by inhibition of kinase activity selected from fibroblast growth factor receptor 1 (FGFR1), fibroblast growth factor receptor 2 (FGFR2), fibroblast growth factor receptor 3 (FGFR3), and fibroblast growth factor receptor 4 (FGFR4).

### DETAILED DESCRIPTION

In one aspect, the present technology provides the N-oxide of 3-(2,6-Dichloro-3,5-dimethoxy-phenyl)-1-{6-[4-(4-ethyl-piperazin-1-yl)-phenylamino]-pyrimidin-4-yl}-1-methyl-urea and pharmaceutically acceptable salts thereof. In some embodiments, the N-oxide is a compound of formula I: 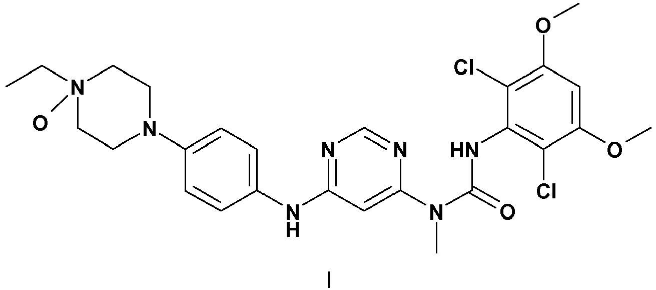 or a pharmaceutically acceptable salt thereof. Surprisingly, it has been found that the compound of formula I may have lower cardiovascular toxicity than the non-oxidized form from which it is derived (i.e., 3-(2,6-Dichloro-3,5-dimethoxy-phenyl)-1-{6-[4-(4-ethyl-piperazin-1-yl)-phenylamino]-pyrimidin-4-yl}-1-methyl-urea) while retaining beneficial biological activity.

As used herein, the pharmaceutically acceptable salts are those formed from salt-forming groups having basic or acidic properties. Compounds having at least one basic group or at least one basic radical, for example amino, a secondary amino group or a pyridyl radical, may form acid addition salts, for example with inorganic acids, such as hydrochloric acid, sulfuric acid or a phosphoric acid, or with suitable organic carboxylic, sulfonic, or other organic acids, for example aliphatic mono- or di-carboxylic acids, such as trifluoroacetic acid, acetic acid, propionic acid, glycolic acid, succinic acid, maleic acid, fumaric acid, hydroxymaleic acid, malic acid, tartaric acid, citric acid or oxalic acid, or amino acids such as arginine or lysine, aromatic carboxylic acids, such as benzoic acid, 2-phenoxy-benzoic acid, 2-acetoxy-benzoic acid, salicylic acid, or 4-aminosalicylic acid, aromatic-aliphatic carboxylic acids, such as mandelic acid or cinnamic acid, heteroaromatic carboxylic acids, such as nicotinic acid or isonicotinic acid, aliphatic sulfonic acids, such as methane-, ethane- or 2-hydroxyethanesulfonic acid, or aromatic sulfonic acids, for example benzene-, p-toluene- or naphthalene-2-sulfonic acid. When several basic groups are present mono- or poly-acid addition salts may be formed.

Certain other suitable inorganic acids are, for example, halogen acids. Certain other suitable organic acids include, for example, other carboxylic, phosphonic, sulfonic or sulfamic acids. Such other examples of suitable acids include octanoic acid, decanoic acid, dodecanoic acid, lactic acid, adipic acid, pimelic acid, suberic acid, azelaic acid, amino acids, such as glutamic acid or aspartic acid, methylmaleic acid, cyclohexanecarboxylic acid, adamantanecarboxylic acid, phthalic acid, phenylacetic acid, mandelic acid, cinnamic acid, methane- or ethane-sulfonic acid, 2-hydroxyethanesulfonic acid, ethane-1,2-disulfonic acid, benzenesulfonic acid, 1,5-naphthalene-disulfonic acid, 2-, 3- or 4-methylbenzenesulfonic acid, methylsulfuric acid, ethylsulfuric acid, dodecylsulfuric acid, N-cyclohexylsulfamic acid, N-methyl-, N-ethyl- or N-propyl-sulfamic acid, or other organic protonic acids, such as ascorbic acid.

For the purposes of isolation or purification, as well as in the case where the compound of the technology is used further as an intermediate, it is also possible to use pharmaceutically unacceptable salts, e.g. the picrates. However, only pharmaceutically acceptable, non-toxic salts may be used for therapeutic purposes.

In another aspect, the present technology provides a compound produced by a process comprising contacting a compound of formula II: 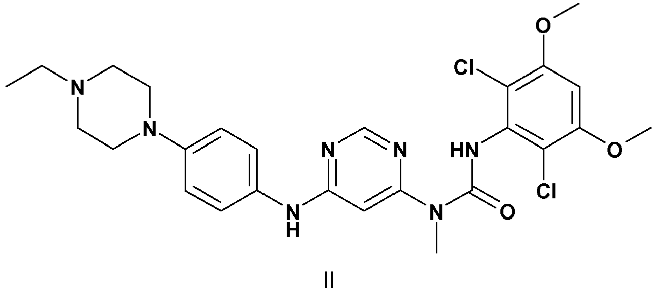 or salts thereof, with an oxidizing agent. In some embodiments of the process, the oxidizing agent is a peroxide, a peracid, or dimethyldioxirane (Me₂CO₂). In one embodiment, the compound produced is a mono-N-oxide, and in others a mixture of N-oxides is produced. In another embodiment, the process further comprises separating the mono-N-oxide from bis-N-oxides and/or other oxides. A variety of methods may be used for the separating, including, without limitation, column chromatography. As used herein, peroxides refer to compounds which include a hydroperoxy (-OOH) moiety and which are not peracids. Examples of peroxides include, without limitation, hydrogen peroxide and urea-hydrogen peroxide, and alkyl hydroperoxides such as tertiary butyl hydroperoxide. As used herein, peracids refer to acids in which the OH moiety of a carboxyl group or an inorganic oxyacid, is replaced by an OOH moiety. Examples of such percarboxylic acids include, without limitation, performic acid and peracetic acid. In one embodiment, the peracid is meta chloroperbenzoic acid (mCPBA). An example of an inorganic peracid is oxone, which is a salt of persulfuric acid (i.e., a persulfate).

Salts, particularly acid salts, of the compound of formula II are also useful for producing the compounds of the present technology according to the processes disclosed herein. Acid salts useful for this purpose can be prepared from acids as described above, and may be, but need not be, pharmaceutically acceptable acid salts. Thus, in some embodiments, the salt of the compound of formula II is a pharmaceutically acceptable salt. In other embodiments, the product produced by the process is a pharmaceutically acceptable salt.

In another aspect, the present technology provides a method of preparing the N-oxide of the present technology comprising contacting a compound of formula II (as shown above) or a salt thereof with an oxidizing agent to provide the compounds of the present technology, including the compound of formula I or salts thereof or pharmaceutically acceptable salts thereof. In some embodiments, the oxidizing agent is a peroxide or a peracid. A variety or peroxides and peracids, including percarboxylic acids and inorganic peracids, as described above, are useful in the methods of the present technology. In one embodiment, the oxidizing agent is meta chloroperbenzoic acid (mCPBA). In another embodiment, the method is performed in a solvent. A variety of solvents, which are stable under the conditions of the contacting step are useful. In certain embodiments, the solvent contains an acid, such as, and without limitation, acetic acid. In some embodiments, the reaction is performed using reactants (the compound of formula II and the oxidizing agent) and one or more solvents that are substantially free of water.

Salts, particularly acid salts, of the compound of formula II are also useful according to the methods of the present technology. Acid salts useful for this purpose can be prepared from acids described above, and additionally, need not be pharmaceutically acceptable acid salts.

In certain embodiments, the oxidizing agent contacted is present in an amount from about 1 equivalent to about 5 equivalents, about 2 equivalents to about 4 equivalents, and about 3 equivalents, with respect to the molar amount of the compound of formula II or an acid salt of the compound of formula II. In certain embodiments, the reactants are reacted or contacted from about 0.3 h to about 3 h, from about 0.6 h to about 2 h, or about 1 h. In some embodiments, the reactants are reacted at a temperature in the range of about -5°C to about 15°C, about 0°C to about 10°C, or about 5°C. On of skill in the art will appreciate upon reading this disclosure that certain other steps may be performed, for example, separating the N-oxide of the present technology from other N-oxides. Such separation can be performed by a variety of methods, including, without limitation, column chromatographic separation.

In another aspect, the present technology provides compositions comprising the compound, or a pharmaceutically acceptable salt thereof, of the present technology, and a pharmaceutically acceptable carrier, excipient, or diluent. In certain embodiments, the compositions are pharmaceutical compositions.

The compounds of the present technology may be used, for example, for the preparation of pharmaceutical compositions that comprise a therapeutically effective amount of a compound of the present technology or a pharmaceutically acceptable salt thereof, as active ingredient together or in admixture with a significant amount of one or more inorganic or organic, solid or liquid, pharmaceutically acceptable carriers, excipients, and/or diluents.

As used herein, a "therapeutically effective amount" refers to an amount of the compound, a pharmaceutically acceptable salt thereof, or compositions including them, that alleviates or ameliorates, in whole or in part, symptoms associated with the disorder or disease treated, or slows or halts of further progression or worsening of its symptoms, or prevents or provides prophylaxis for the disease or disorder in a subject at risk for developing the disease or disorder.

A "subject" is any warm blooded animal that can benefit from the administration of the compound, a pharmaceutically acceptable salt thereof, or compositions including them, as disclosed herein. In some embodiments, the subject is a mammal, for example, a human, a primate, a dog, a cat, a horse, a cow, a pig, a rodent, such as for example a rat or mouse. Typically, the mammal is a human.

The present technology relates also to a pharmaceutical composition that is suitable for administration to a subject animal, especially a human (or to cells or cell lines derived from a warm blooded animal, especially a human, e.g. lymphocytes), for the treatment or, in a broader aspect of the technology, prevention of (= prophylaxis against) or amelioration of a disease and/or its symptoms that respond to inhibition of protein kinase activity. In one embodiment, the protein kinase is a tyrosine kinase. In another embodiment, the protein kinase is FGFR1, FGFR2, FGFR3, or FGFR4,.

Compositions for enteral administration, such as nasal, buccal, rectal or, especially, oral administration, and for parenteral administration, such as intravenous, intramuscular or subcutaneous administration, to a subject, especially humans, are provided in accordance with the present technology. The compositions comprise the active ingredient alone or, together with a pharmaceutically acceptable carrier. The dosage of the active ingredient depends upon the disease to be treated and upon the species, its age, weight, and individual condition, the individual pharmacokinetic data, and the mode of administration.

a The technology relates also to pharmaceutical compositions for use in a method for the prophylactic or, especially, therapeutic management of the human or animal body, to a process for the preparation thereof (especially in the form of compositions for the treatment of tumors).

The pharmaceutical compositions comprise from approximately 1% to approximately 95% active ingredient, single-dose administration forms comprising in certain embodiment from approximately 20% to approximately 90% active ingredient and forms that are not of single-dose type comprising in certain embodiment from approximately 5% to approximately 20% active ingredient. Unit dose forms are, for example, coated and uncoated tablets, ampoules, vials, suppositories, or capsules. Further dosage forms are, for example, ointments, creams, pastes, foams, tinctures, sprays, etc. Examples are capsules containing from about 0.05 g to about 1.0 g active ingredient.

The pharmaceutical compositions of the present technology are prepared in a manner known per se, for example by means of conventional mixing, granulating, coating, dissolving or lyophilizing processes.

In certain embodiments, solutions of the active ingredient are used, and also suspensions or dispersions, especially isotonic aqueous solutions, dispersions or suspensions which, for example in the case of lyophilized compositions comprising the active ingredient alone or together with a carrier can be made up before use. The pharmaceutical compositions may be sterilized and/or may comprise excipients, for example preservatives, stabilizers, wetting agents and/or emulsifiers, solubilizers, salts for regulating osmotic pressure and/or buffers and are prepared in a manner known per se, for example by means of conventional dissolving and lyophilizing processes. Such solutions or suspensions may comprise viscosity-increasing agents or solubilizers, such as sodium carboxymethylcellulose, carboxymethylcellulose, dextran, polyvinyl pyrrolidone or gelatin.

Suspensions in oil comprise as the oil component the vegetable, synthetic or semisynthetic oils customary for injection purposes. There may be mentioned as such especially liquid fatty acid esters that contain as the acid component a long-chained fatty acid having from 8 to 22, especially from 12 to 22, carbon atoms, for example lauric acid, tridecylic acid, myristic acid, pentadecylic acid, palmitic acid, margaric acid, stearic acid, arachidic acid, behenic acid or corresponding unsaturated acids, for example oleic acid, elaidic acid, erucic acid, brasidic acid or linoleic acid, if desired with the addition of antioxidants, for example vitamin E, ß-carotene or 3,5-di-tert-butyl-4-hydroxytoluene. The alcohol component of those fatty acid esters has a maximum of 6 carbon atoms and is a mono- or poly-hydroxy, for example a mono-, di- or tri-hydroxy, alcohol, for example methanol, ethanol, propanol, butanol or pentanol or the isomers thereof, but especially glycol and glycerol. The following examples of fatty acid esters are therefore to be mentioned: ethyl oleate, isopropyl myristate, isopropyl palmitate, "Labrafil M 2375" (polyoxyethylene glycerol trioleate, Gattefossé, Paris), "Miglyol 812" (triglyceride of saturated fatty acids with a chain length of C₈ to C₁₂, Hüls AG, Germany), but especially vegetable oils, such as cottonseed oil, almond oil, olive oil, castor oil, sesame oil, soybean oil and more especially groundnut oil.

Injection compositions are prepared in customary manner under sterile conditions; the same applies also to introducing the compositions into ampoules or vials and sealing the containers.

Pharmaceutical compositions for oral administration can be obtained by combining the active ingredient with solid carriers, if desired granulating a resulting mixture, and processing the mixture, if desired or necessary, after the addition of appropriate excipients, into tablets, dragée cores or capsules. It is also possible for them to be incorporated into plastics carriers that allow the active ingredients to diffuse or be released in measured amounts.

Suitable carriers are especially fillers, such as sugars, for example lactose, saccharose, mannitol or sorbitol, cellulose preparations and/or calcium phosphates, for example tricalcium phosphate or calcium hydrogen phosphate, and binders, such as starch pastes using for example corn, wheat, rice or potato starch, gelatin, tragacanth, methylcellulose, hydroxypropylmethylcellulose, sodium carboxymethylcellulose and/or polyvinylpyrrolidone, and/or, if desired, disintegrators, such as the above-mentioned starches, and/or carboxymethyl starch, crosslinked polyvinylpyrrolidone, agar, alginic acid or a salt thereof, such as sodium alginate.

Excipients are especially flow conditioners and lubricants, for example silicic acid, talc, stearic acid or salts thereof, such as magnesium or calcium stearate, and/or polyethylene glycol. Dragée cores are provided with suitable, optionally enteric, coatings, there being used, *inter alia,* concentrated sugar solutions which may comprise gum arabic, talc, polyvinylpyrrolidone, polyethylene glycol and/or titanium dioxide, or coating solutions in suitable organic solvents, or, for the preparation of enteric coatings, solutions of suitable cellulose preparations, such as ethylcellulose phthalate or hydroxypropylmethylcellulose phthalate.

Capsules are dry-filled capsules made of gelatin and soft sealed capsules made of gelatin and a plasticizer, such as glycerol or sorbitol. The dry-filled capsules may comprise the active ingredient in the form of granules, for example with fillers, such as lactose, binders, such as starches, and/or glidants, such as talc or magnesium stearate, and if desired with stabilizers. In soft capsules the active ingredient is dissolved or suspended in suitable oily excipients, such as fatty oils, paraffin oil or liquid polyethylene glycols, it being possible also for stabilizers and/or antibacterial agents to be added. Dyes or pigments may be added to the tablets or dragée coatings or the capsule casings, for example for identification purposes or to indicate different doses of active ingredient.

Tablet cores can be provided with suitable, optionally enteric, coatings through the use of, inter alia, concentrated sugar solutions which may comprise gum arabic, talc, polyvinylpyrrolidone, polyethylene glycol and/or titanium dioxide, or coating solutions in suitable organic solvents or solvent mixtures, or, for the preparation of enteric coatings, solutions of suitable cellulose preparations.

Pharmaceutical compositions for oral administration also include hard capsules consisting of gelatin, and also soft, sealed capsules consisting of gelatin and a plasticizer. The hard capsules may contain the active ingredient in the form of granules, for example in admixture with fillers, binders, and/or glidants, and optionally stabilizers. In soft capsules, the active ingredient is dissolved or suspended in suitable liquid excipients, to which stabilizers and detergents may also be added.

Pharmaceutical compositions suitable for rectal administration are, for example, suppositories that consist of a combination of the active ingredient and a suppository base.

For parenteral administration, aqueous solutions of an active ingredient in water-soluble form, for example of a water-soluble salt, or aqueous injection suspensions that contain viscosity-increasing substances, for example sodium carboxymethylcellulose, sorbitol and/or dextran, and, if desired, stabilizers, are especially suitable. The active ingredient, optionally together with excipients, can also be in the form of a lyophilizate and can be made into a solution before parenteral administration by the addition of suitable solvents.

Solutions which are used, for example, for parenteral administration, can also be employed as infusion solutions.

The compound of the technology can be administered as such or especially in the form of pharmaceutical compositions, prophylactically or therapeutically, in an amount effective against the said diseases, to a subject, for example a human, requiring such treatment. In the case of an individual having a bodyweight of about 70 kg the daily dose administered is from approximately 0.05 g to approximately 5 g, or from approximately 0.25 g to approximately 1.5 g, of a compound of the present technology.

The present technology relates to the use of the compound, or a pharmaceutically acceptable salt thereof, of the present technology, as such or in the form of a pharmaceutical composition with at least one pharmaceutically acceptable carrier for the therapeutic and also prophylactic management of one or more of the diseases mentioned above, for example, and without limitation, a disease which responds to an inhibition of a protein kinase, especially a neoplastic or tumor disease, especially solid tumor, more especially those cancers in which FGFR kinases are implicated including breast cancer, gastric cancer, lung cancer, cancer of the prostate, bladder cancer and endometrial cancer. Further cancer includes kidneys, liver, adrenal glands, stomach, ovaries, colon, rectum, pancreas, vagina or thyroid, sarcoma, glioblastomas and numerous tumours of the neck and head, as well as leukemias and multiple myeloma. In a further aspect, the present invention related to the use of the compound of the present invention for the treatment of a warm-blooded animal having a disorder mediated by the fibroblast growth factor receptor (FGFR), in particular 8p11 myeloproliferative syndrome (EMS), pituitary tumors, retinoblastoma, synovial sarcoma, chronic obstructive pulmonary disease (COPD), seborrheic keratosis, obesity, diabetes and related disorders, autosomal dominant hypophosphatemic Rickets (ADHR), X-chromosome linked hypophosphatemic rickets (XLH), tumor-induced osteomalacia (TIO) and fibrous dysplasia of the bone (FD) as well as to a method of promoting localized neochondrogenesis, hepatocellular carcinoma, lung cancer, especially pulmonary adenocarcinoma, oral squameous cell carcinoma or esophageal squameous cell carcinoma, or any combination of two or more such diseases.

Dose quantity, composition, and preparation of pharmaceutical compositions (medicines) which are to be used are described above.

The compound of the present technology and pharmaceutically acceptable salts thereof may also be used to advantage in combination with other antiproliferative agents. Such antiproliferative agents include, but are not limited to aromatase inhibitors, antiestrogens, topoisomerase I inhibitors, topoisomerase II inhibitors, microtubule active agents, alkylating agents, histone deacetylase inhibitors, farnesyl transferase inhibitors, COX-2 inhibitors, MMP inhibitors, mTOR inhibitors, antineoplastic antimetabolites, platin compounds, compounds decreasing the protein kinase activity and further anti-angiogenic compounds, gonadorelin agonists, anti-androgens, bengamides, bisphosphonates, antiproliferative antibodies and temozolomide (TEMODAL®).

Means for determining protein kinase inhibitory activity of other compounds and methods for treating, preventing or ameliorating protein kinase mediated diseases, particularly tyrosine kinase mediated diseases, by administering other active agents are described in PCT App. Pub. No. WO 06/000420 and can be adapted by one of skill in the art for the treatment methods of the present technology upon reading this disclosure.

The present technology, thus generally described, will be understood more readily by reference to the following examples, which are provided by way of illustration and are not intended to be limiting of the present technology.

### EXAMPLES

The following abbreviations are used throughout the present disclosure with respect to chemical and biological terminology:
- AcOH: Acetic acid
- DCM: Dichloromethane
- h: Hour(s)
- P: Partition coefficient
- mCPBA: m-Cloroperbenzoic acid
- MeOH: Methanol
- mL: Milliliter(s)
- R_{f}: Ratio of fronts (TLC)
- TLC: Thin layer chromatography
- TFA: Trifluoroacetic acid
- t_{R}: Retention time

### Synthesis of the N-oxide of 3-(2,6-Dichloro-3,5-dimethoxy-phenyl)-1-{6-[4-(4-ethyl-piperazin-1-yl)-phenylamino]-pyrimidin-4-yl-}-1methyl-urea (1)

mCPBA (55%, 253 mg, 0.81 mmol) was added portion-wise over 15 min to a cold (5°C) solution of 3-(2,6-Dichloro-3,5-dimethoxy-phenyl)-1-{6-[4-(4-ethyl-piperazin-1-yl)-phenylamino]-pyrimidin-4-yl}-1-methyl-urea (Compound 2; 500 mg, 0.89 mmol, 1.1 equiv) in DCM (70 mL) and AcOH (1.5 mL). The resulting mixture was stirred for 1 h at 5°C and diluted with DCM/saturated aqueous solution of NaHCO₃. The aqueous layer was separated and extracted with DCM. The combined organic extracts were washed with water and a saturated solution of NaCl in water, dried (Na₂SO₄), filtered and concentrated. The residue was purified by silica gel column chromatography (DCM/MeOH/ aqueous NH₃, 89:10:1) followed by trituration in diethyl ether to provide 230 mg of the title compound (Compound 1) as a white solid: ESI-MS: m/z 576.0 [M+H]⁺; t_{R}= 3.57 min; TLC: R_{f} = 0.23 (DCM/MeOH/ NH₃^{aq}, 89:10:1). ¹H NMR (400 MHz, d6-dmso>) δ ppm 1.24 (t, 3 H) 2.93 (d, 2 H) 3.19 (q, 2 H) 3.26 - 3.44 (m, 9 H) 3.92 (s, 6 H) 6.42 (s, 1 H) 6.88 (s, 1 H) 6.96 (m, 2 H) 7.43 (m, 2 H) 8.37 (s, 1 H) 9.53 (s, 1 H) 12.03 (s, 1 H). Analytical HPLC conditions were as follows: linear gradient 20-100% solvent A in 5 min + 1.5 min 100% solvent A; detection at 215 nm; flow rate 1 mL/min at 30°C; column: Nucleosil 100-3 C18 (70 × 4.0 mm); solvent A = CH₃CN + 0.1 % TFA, solvent B = H₂O + 0.1 % TFA.

Certain physicochemical properties of the compounds, as measured, are provided below:

**Table 1**

| Physicochemical property | Compound 2 | Compound 1 |
|---|---|---|
| pKa1/pKa2* | 8.2 /3.4 | 4.5/3.2 |
| Solubility in buffer (pH 1) | 2 mM | 0.7 mM |

| | | |
|---|---|---|
| * Ionization constants for acidity | | |

### Demonstration of Enhanced Metabolic Stability of Compound 1

The metabolism of drugs occurs mainly in the liver, which contains a wide variety of metabolic enzymes at concentrations higher than those observed in other organs. Metabolic stability or clearance (CL), particularly hepatic clearance (CLₕ), is a factor in determining drug concentration in blood. Generally, the higher the metabolic stability of a compound is, the lower the clearance of the compound.

The metabolic stability of each of Compounds 1 and 2 were determined at a concentration of 1 µM in mouse, rat, dog, monkey and human hepatic microsomal preparations. The compound tested, microsomal protein, and co-factors were combined, in duplicate, and incubated under appropriate conditions. Aliquots were removed at 0, 10 and 30 min, centrifuged and the supernatants analyzed by LC/MS/MS. Based on the percentage of the test compound remaining relative to the 0 min time point, the *in vitro* elimination rate constant, *in vitro* half-life (t_{1/2}) and *in vitro* intrinsic clearance (CLᵢₙₜ) parameters were calculate which allow for predicting the CLₕ values. The results are tabulated below and demonstrate that Compound 1 has a higher metabolic stability than Compound 2.

**Table 2: In vitro metabolic stability of Compound 2 in liver microsomes**

| Species | t_{1/2} (min) | CLᵢₙₜ (µL·min⁻¹mg⁻¹) | CLᵢₙₜ Rank* |
|---|---|---|---|
| Mouse | 10.3 | 134 | medium |
| Rat | 15.4 | 90.3 | medium |
| Dog | 3.5 | 395 | high |
| Monkey | 3.8 | 364 | high |
| Human | 35.2 | 39.4 | low |

**Table 3: In vitro metabolic stability of Compound 1 in liver microsomes**

| Species | t_{1/2} (min) | CLᵢₙₜ (µL·min⁻¹mg⁻¹) | CLᵢₙₜ Rank* |
|---|---|---|---|
| Mouse | 55.2 | 25.1 | low |
| Rat | 46.8 | 29.6 | low |
| Dog | 30.1 | 46.1 | low |
| Monkey | 35.7 | 38.9 | low |
| Human | 37.4 | 37.1 | low |

| | | | |
|---|---|---|---|
| * Low: CLᵢₙₜ < 50 µL·min⁻¹mg⁻¹; medium: 50 < CLᵢₙₜ < 150 µL·min⁻¹mg⁻¹; high: CLᵢₙₜ > 150 µL·min⁻¹mg⁻¹ | | | |

### Demonstration of Lower Potassium Channel Inhibition by Compound 1 Compared to Compound 2 by In-Vitro Electrophysiology

The ability of Compounds 1 and 2 to inhibit the hERG (potassium channel) activity was tested using a radioligand binding assay in which test compounds competed for [³H]dofetilide binding to a membrane preparation of HEK293 cell membranes stably transfected with hERG channels. The IC₅₀ value determined for Compound 2 was 3.0 µM, and for Compound 1 was 10.2 µM. The results demonstrate that, based on its significantly lower activity in inhibiting hERG (potassium channel), Compound 1 may show lower cardiovascular toxicity than Compound 2 when administered to subjects. Cardiovascular toxicity of compounds is not predictable, and the reduced inhibition of potassium channels exhibited by Compound 1 compared to Compound 2 is therefore surprising and unexpected.

### Cell Permeability

The permeability of Compounds 1 and 2 across the intestinal barrier and the involvement of drug efflux transporters such as P-glycoprotein (P-gp, MDR-1) were assessed *in vitro* using the Caco-2 cell line. Caco-2 cells are a human colonic adenocarcinoma cell line used to demonstrate drug absorption, and the role of non-passive diffusion processes in drug transport. Caco-2 cells were seeded on PET (polyethylene terephthalate) filters in a 96 well format and cultured for 18-25 days to develop monolayers. Test compound solutions (10 µM in transport buffer) were added to either the apical (A) or basolateral (B) side of the Caco-2 cell monolayer to measure the membrane permeability from the A to B compartments [Papp(A-B)] or from the B to A compartments [Papp(B-A]. The assay was carried out in HBSS (Hank's Balanced Salts) buffer, pH 7.4 (for both sides) for 120 min at 37 °C. Samples were taken from apical or basolateral compartments at scheduled times (0 min and 120 min) and quantified by LC/MS/MS. The results are presented in Table 4.

**Table 4: Permeability of Compounds 1 and 2 across Caco-2 cell monolayers**

| | Pₐₚₚ | | B-A/A-B | Permeability* Ranking | Mechanism** |
|---|---|---|---|---|---|
| | A-B (10⁻⁶ cm/sec) | B-A (10⁻⁶ cm/sec) | | | |
| Compound 2 | 1.69 | 1.59 | 0.94 | Medium | Passive transcellular |
| Compound 1 | 0.47 | 9.51 | 20.21 | Low | Efflux |

| | | | | | |
|---|---|---|---|---|---|
| * High: A-B > 5x10⁻⁶ cm/sec; medium: 1x·10⁻⁶ < A-B < 5x·10⁻⁶ cm/sec; low A-B < 1x·10⁻⁶ cm/sec. ** Passive transcellular: B-A/A-B < 2 and clog P ≥ 1 (clogP = calculated octanol/water distribution coefficient). Efflux: B-A/A-B ≥ 3. | | | | | |

## Claims

1. A compound of formula I: 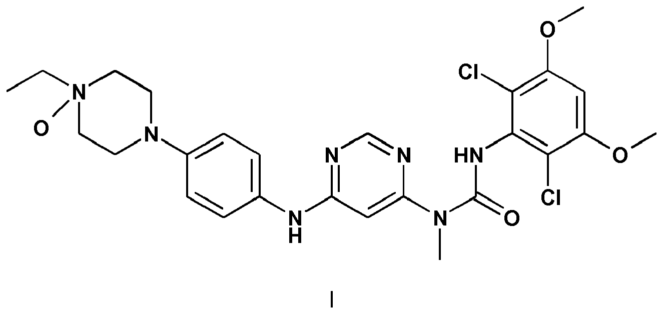 or a pharmaceutically acceptable salt thereof.

2. A composition comprising the compound or pharmaceutically acceptable salt of the compound of claim 1 and a pharmaceutically acceptable carrier, excipient, or diluent.

3. A method of synthesizing the compound of claim 1 comprising contacting a compound of formula II: 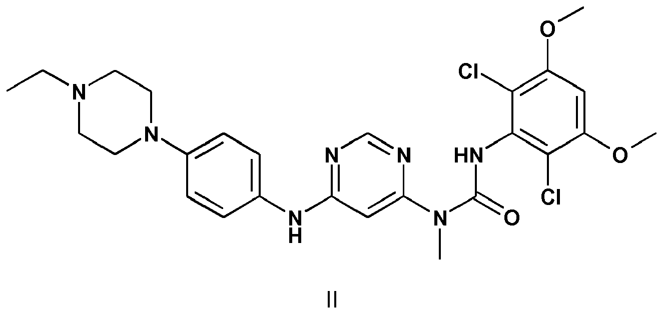 or a salt thereof with an oxidizing agent to provide the compound of claim 1.

4. The method of claim 3, wherein the oxidizing agent is a peroxide or a peracid.

5. The method of claim 3, wherein the oxidizing agent is meta chloroperbenzoic acid (mCPBA).

6. The compound of claim 1 or the composition of claim 2 for use in treating a disease selected from the group consisting of breast cancer, gastric cancer, lung cancer, cancer of the prostate, bladder cancer and endometrial cancer in a subject.

7. The compound of claim 1 for use in combination with one or more cytostatic or cytotoxic compounds in treating a disease selected from the group consisting of breast cancer, gastric cancer, lung cancer, cancer of the prostate, bladder cancer and endometrial cancer in a subject.

## Patentansprüche

1. Verbindung der Formel I: 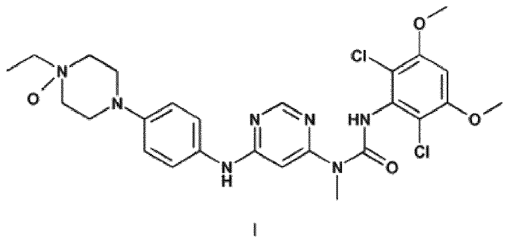 oder ein pharmazeutisch annehmbares Salz davon.

2. Zusammensetzung, umfassend die Verbindung oder ein pharmazeutisch annehmbares Salz der Verbindung nach Anspruch 1 und einen pharmazeutisch annehmbaren Träger, Hilfsstoff oder ein pharmazeutisch annehmbares Verdünnungsmittel.

3. Verfahren zur Synthese der Verbindung nach Anspruch 1, umfassend das Kontaktieren einer Verbindung der Formel II: 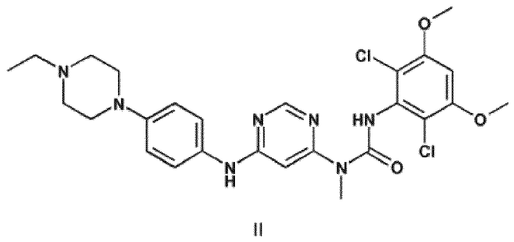 oder eines Salzes davon mit einem Oxidationsmittel zur Bereitstellung der Verbindung nach Anspruch 1.

4. Verfahren nach Anspruch 3, wobei das Oxidationsmittel ein Peroxid oder eine Persäure ist.

5. Verfahren nach Anspruch 3, wobei das Oxidationsmittel meta-Chlorperbenzoesäure (mCPBA) ist.

6. Verbindung nach Anspruch 1 oder Zusammensetzung nach Anspruch 2 zur Verwendung bei der Behandlung einer Erkrankung, ausgewählt aus der Gruppe bestehend aus Brustkrebs, Magenkrebs, Lungenkrebs, Prostatakrebs, Blasenkrebs und Endometriumkrebs bei einem Probanden.

7. Verbindung nach Anspruch 1 zur Verwendung in Kombination mit einer oder mehreren zytostatischen oder zytotoxischen Verbindungen bei der Behandlung einer Erkrankung, ausgewählt aus der Gruppe bestehend aus Brustkrebs, Magenkrebs, Lungenkrebs, Prostatakrebs, Blasenkrebs und Endometriumkrebs bei einem Probanden.

## Revendications

1. Composé de formule I : 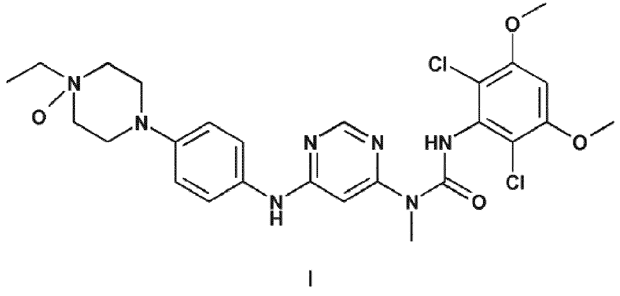 ou sel pharmaceutiquement acceptable de celui-ci.

2. Composition comprenant le composé ou le sel pharmaceutiquement acceptable du composé de la revendication 1 et un véhicule, un excipient ou un diluant pharmaceutiquement acceptable.

3. Procédé de synthèse du composé de la revendication 1 comprenant la mise en contact d'un composé de formule II : 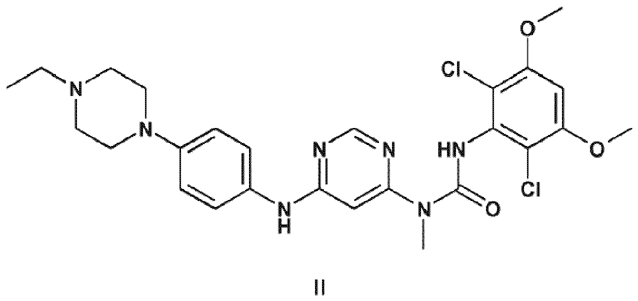 ou sel de celui-ci avec un agent oxydant pour obtenir le composé de la revendication 1.

4. Procédé selon la revendication 3, dans lequel l'agent oxydant est un peroxyde ou un peracide.

5. Procédé selon la revendication 3, dans lequel l'agent oxydant est un acide méta chloroperbenzoïque (mCPBA).

6. Composé selon la revendication 1 ou composition selon la revendication 2, servant à traiter une maladie choisie dans l'ensemble constitué du cancer du sein, du cancer de l'estomac, du cancer du poumon, du cancer de la prostate, du cancer de la vessie et du cancer de l'endomètre chez un sujet.

7. Composé selon la revendication 1, servant en combinaison avec au moins un composé cytostatique ou cytotoxique pour traiter une maladie choisie dans l'ensemble constitué du cancer du sein, du cancer de l'estomac, du cancer du poumon, du cancer de la prostate, du cancer de la vessie et du cancer de l'endomètre chez un sujet.
